# EUROPEAN PATENT SPECIFICATION

(11) **EP 0 428 352 B1**
(45) Date of publication and mention of the grant of the patent: **09.08.1995**
(21) Application number: 90312313.1
(22) Date of filing: 12.11.1990
(51) Int. Cl.: A61K 31/19, A61K 31/195, A61K 31/54, A61K 47/12, A61K 47/14

(54) **Anti-inflammatory compositions for external use**
Entzündungshemmende Arzneimittel zur äusserlichen Anwendung
Compositions antiinflammatoires à utilisation externe

(30) Priority: 14.11.1989 AR 315433
(43) Date of publication of application: 22.05.1991
(73) Proprietor: Beta Pharmaceuticals Co., 1201 Montevideo (UY)
(72) Inventor: Stefano, Francisco José Evaristo, Buenos Aires (AR); Nowogrodski, Jorge Adrián, Buenos Aires (AR); Carrara, Darîo Norberto Ramón, Hurlingham, Buenos Aires (AR)
(74) Representative: Pearce, Anthony Richmond

(56) References cited:
- US-A- 4 837 025
- DATABASE: WPIL, accession no. 89-059211 [08], Derwent Publications Ltd, London,GB; & JP-A-01 013 020 (SHISEIDO) 17-01-1989
- STN INTERNATIONAL INFORMATION SERVICES DATA BASE: CHEMICAL ABSTRACTS, vol. 109,no. 6, abstract no. 43457r; & JP-A-62 240 614 (SEKISUI) 21-10-1987
- DATABASE: WPIL, accession no. 84-084432 [14], Derwent Publications Ltd, London,GB; & JP-A-59 033 211 (SATO) 23-02-1984
- DATABASE: WPIL, accession no. 86-289030 [44], Derwent Publications Ltd, London,GB; & JP-A-61 212 516 (TOYOBO) 20-09-1986

## Description

This invention relates to anti-inflammatory compositions.

### Background art

Medical practice uses various anti-inflammatory compositions, which are divided into two main groups known as non-steroid anti-inflammatory compositions (NSAI) and steroid anti-inflammatory compositions (SAI). The first category contains several well known compositions, eg pyroxicam and naproxen. Dexamethasone, cortisone, prednisolone and hydrocortisone fall within the group of steroid anti-inflammatories.

Inflammatory reaction is known to be one of the body's defence mechanisms. It is characterised by high temperature, pain and oedema mediated by the prostaglandins (PGF_{1α}, PGD₂, PGE_{2α}, etc) which are fatty substances produced in most tissue at the end of a complex enzymatic process from phospholipids which are present in cellular membranes. The phospholipids are broken down by phospholipase A₂, and arachidonic acid is produced and degraded either to leucotriene by one metabolic route or to prostaglandins by another metabolic route. These compounds are not stored in the cells as they are synthesised as an immediate response to chemical or mechanical lesions of the cell. They trigger certain manifest reactions which are concomitant with fever or allergy.

The biological properties of both the non-steroid and steroid anti-inflammatories (NSAI and SAI respectively) are described in terms of their ability to block the synthesis of prostaglandins. Steroid anti-inflammatories (cortisone) inhibit the enzymatic synthesis of arachidonic acid by blockage of the initial step, phospholipase A₂ breakdown of phospholipids, whilst the NSAI intervene at the next stage by inhibiting the conversion of arachidonic acid into prostaglandins.

Anti-inflammatory compositions are administered orally, by injection, rectally or topically according to circumstance.

According to current knowledge, NSAI are only administered orally or intramuscularly, or even rectally, except when dealing with acetylsalicylic acid or its salts and esters, for general application in the form of ointments, creams etc. There are even pyroxicam preparations for external use (creams and salves,eg Dipironal^{R}).

Topical application for most anti-inflammatory agents is restricted due to their reduced rate of permeation through the skin, a parameter that depends, as has been shown, on a number of factors that converge to adverse effect: physical and chemical properties of the compound, properties of the vehicle and the areas of the skin to which it is applied (abundance of sebaceous glands, hair follicles, thickness of the keratin layer etc.)

These factors have been overcome in certain cases by means of additives that increase the rate of permeation ("enhancers"). These additives are thought to modify the tissue and physiological structure of intra- and inter-cellular exchange, the net result of which is increased rate of permeation. Proposed additives are: oleic acid and its salts, low alkyl esters associated with alkenes (C₁-C₃ (EPA 0267617); oleic acid and/or 2-ethyl-3-hexanodiol (WO 87/03490), 2H-azepine-2-ona pursuant to EPA 0251.425 etc.

Administration of these additives has led to the development of many and varied forms of percutaneous administration for nitroglycerine, estradiol, anorexigens etc. and even for analgesics and many current anti-inflammatory drugs, by the use of dressing or self-adhesive film (EPA 026717, supra)

WPIL, accession no. 86-289030[49], Derwent Publications Ltd, London, GB (JP-A-61 212 516) discloses an anti-inflammatory ointment for external use in which pyroxicam (an NSAI) is used as the active ingredient and in which ointment a skin-absorbing promoting agent (preferably N-methylpyrrolidone, urea, dimethylsulphoxide, a pyrrolidone derivative, free fatty acids, 1-dodecylazacycloheptan-2-one, amines and propylene glycol) is included. WPIL, accession no. 89-059211 [08], Derwent Publications Ltd, London, GB (JP-A-01 013 020) discloses an emulsifying drug composition comprising diclofenac sodium salt, a fatty acid (especially isostearic acid, oleic acid or linoleic acid), and a carboxylic acid dialkyl ester, these latter two components being used to improve the stability of the emulsion.

We have discovered that it is possible to use pharmaceutical ingredients for external use in the form of gels, formulated with anti-inflammatory agents and specific enhancers.

### Summary of the invention

In one aspect, the present invention resides in a pharmaceutical composition for external use and which has, as an active ingredient, a non-steroid anti-inflammatory compound (NSAI), characterised in that said active ingredient is a compound selected from:
i) 4-hydroxy-2-methyl-N-2-pyridinyl-2H-1,2-benzothiazine-3-carboxamide-1,1-dioxide (pyroxicam) of the formula: 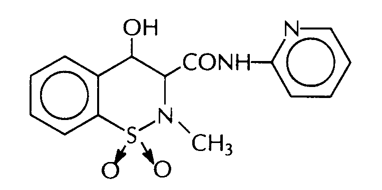
ii) alpha-(6-methoxy)naphthyl-propionic acid (naproxen) of the formula: 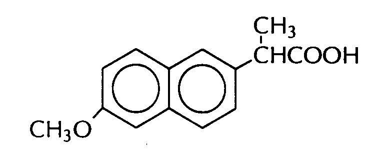
iii) alpha-(4-isobutyl)phenyl-propionic acid (ibuprofen) of the formula: 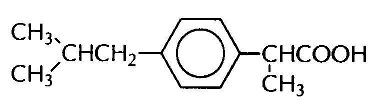
iv) a pharmaceutically acceptable salt of compound i), ii) or iii),
v) the diethylammonium salt of 2-(2,6-dichloroanilino)phenyl-acetic acid (diclofenac) of the formula: 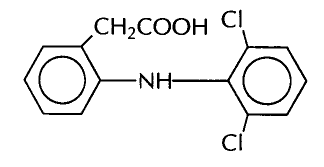 in that a pharmaceutically effective amount of said active ingredient is present in a pharmaceutically acceptable vehicle that includes, as a percutaneous penetration enhancer of said active ingredient, at least one of (C₆-C₁₈) fatty acids and alkyl and alkenyl esters thereof, and in that the pharmaceutical composition is a gel.

In another aspect, the present invention resides in a process for the preparation of a pharmaceutical composition as defined in the last preceding paragraph, which is characterised in that it consists of forming a homogenous gel of a non-steroid anti-inflammatory agent selected from pyroxicam, ibuprofen, diethylammonium salt of dichlofenac and naproxen in a pharmaceutically acceptable vehicle that includes, as an enhancer of percutaneous penetration, at least one of (C₆-C₁₈) fatty acids and their alkyl or alkenyl esters.

### Description of the drawings

Figure 1 is a diagram of the side view of a diffusion chamber device used to test the rate of permeation by compounds tested on a skin sample, as described below, which is given the identification number 10, and
Figure 2 shows the same device, divided into its main constituent parts.

Referring to the drawings, the diffusion chamber device consists of two vertically arranged compartments 11 and 12. The upper compartment 11 is a feed compartment and is open to atmosphere at its top. The lower compartment 12 is a receptor compartment which is maintained at 37°C by a thermostatically heated aluminium block. The compartments 11 and 12 have respective mutually-facing annular flanges 13 and 14 between which a skin sample 19 to be subjected to testing is clamped by means of a clamp 17.

For the permeation tests, the NSAI solutions are loaded into the upper compartment 11 and the permeated solution is collected in the receptor compartment 12 and subjected to agitation using a star-head magnet 20 driven by a magnetic stirrer disposed under the compartment 12. The samples are extracted via a sampling port 18.

### Detailed description of the invention

The present invention concerns improvements observed in the percutaneous absorption of anti-inflammatories, drugs dispersed or dissolved in the form of gels when a non-polar substance such as fatty acids or small amounts of their esters are added.

The tested substances are fatty acids having the general formula CₙH₂ₙ₊₁ and/or CₙH₂ₙCOOH where n is a whole number from 4 to 30, including its polyunsaturated derivatives (two or more double bonds C=C), its (C₁-C₃₀)alkyl or (C₂-C₃₀)alkenyl esters including its polyunsaturated derivatives (with two or more double bonds C=C).

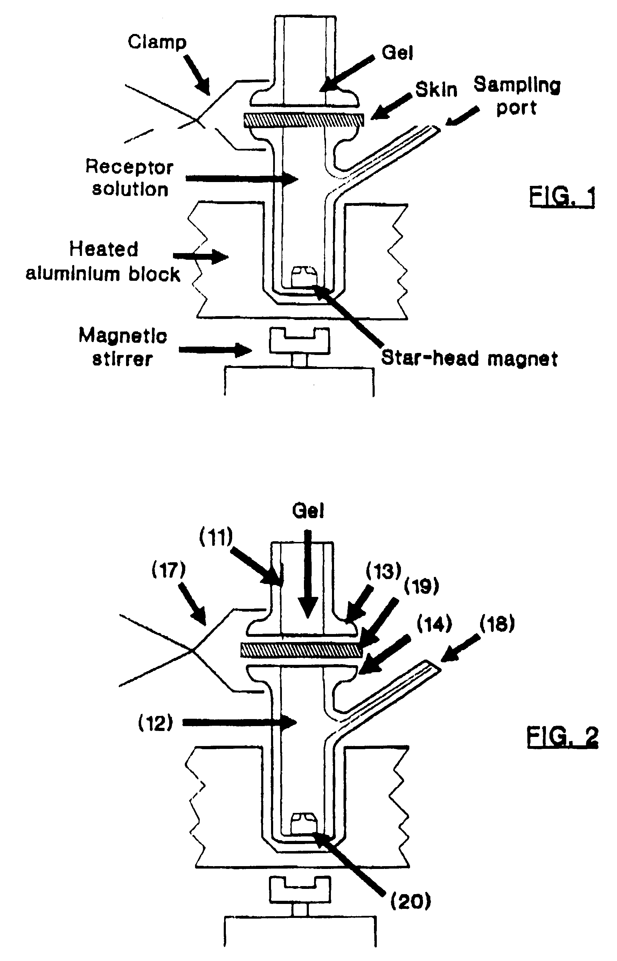

The properties of the pharmaceutical compositions of the present invention were observed and verified in experiments using a glass diffusion chamber device as illustrated in Figure 1.

The skin samples are obtained from the abdomen of female mice of the Swiss strain that have been shaved 72 hours before the experiment. The skin samples are placed over the mouth of the lower tube 12 of the diffusion cell, with the dermis on the side of the recipient solution. The upper tube 11 is placed on the epidermal side of the skin 19; the test sample containing NSAI is added to the upper tube of the cell.

The recipient solution is a phosphate buffer; it is maintained at 34°C and is constantly agitated throughout the experiment (magnetic agitator 20).

The recipient solution sample is withdrawn via side tube 18 at six hours and it is analyzed for the assayed drug.

The results are given as micrograms of permeated drug per unit of surface within a given time span.

In the following Examples, given here by way of illustration, composition preparation on the basis of this invention is described.

### Example 1:

To a mixture of 20g isopropanol, 48.5g distilled water, 12g propylene glycol, 1g carbopol, 7g polyethylene glycol, 3g ethanol, 1g triethanolamine and 5g vaseline is added 1.15g diclofenac (diethylammonium salt); it is then homogenised.

### Example 2:

To the gel prepared as described in Example 1 are also added 4.5g propyleneglycol monolaurate.

### Example 3:

To the mixture prepared as described in Example 1 are added 4.8g oleic acid.

### Example 4:

To the mixture prepared as described in Example 1 are added 4.7g isopropyl myristate.

### Example 5:

To the mixture prepared as described in Example 1 are added 2.5g isopropyl myristate.

### Example 6:

To the mixture prepared as described in Example 1 are added 9.7g isopropyl myristate.

### Example 7:

To the mixture described in Example 1 are added 1.0g ibuprofen and 4.8g isopropyl myristate.

To demonstrate the enhanced permeability of the substances described in Examples 1 to 7, the following experiments were carried out.

### Example 8:

1g of the mixture described in Example 1 is placed in the upper part of the cell shown in Figure 1. The contents of the lower part of the cell is maintained at a pH of 7.4 with a phosphate solution and is heated to 34°C.

### Example 9:

Example 8 is repeated using the mixture of Example 2.

### Example 10:

Example 8 is repeated using the mixture of Example 3.

### Example 11:

Example 8 is repeated using the mixture of Example 4.

### Example 12:

Example 8 is repeated using the mixture of Example 5.

### Example 13:

Example 8 is repeated using the mixture of Example 6.

### Example 14:

Example 8 is repeated using the mixture of Example 7.

### Results

**TABLE 1**

| Examples | 8 | 9 | 10 | 11 | 12 | 13 | 14 |
|---|---|---|---|---|---|---|---|
| Permeation-enhancing additive | - | PGML | AO | MIP | MIP | MIP | MIP |
| Flow of NSAI after 6 hrs (»g/cm²h) | 10 | 27 | 150 | 19 | 30 | 28 | 36 |
| PGML: propylene glycol monolaurate OA: oleic acid IPM: isopropyl myristate. | | | | | | | |

As Table 1 shows, the most efficient enhancer of permeability for diclofenac (diethylammonium salt) was oleic acid 5%, followed by IPM where it is observed that the effect depends of the concentration, up to concentrations of 5%. The effect of propylene glycol monolaurate was approximately 60% of that observed with IPM.

## Claims (Claims for the following Contracting State(s): AT, BE, DE, FR, GB, IT, LU, NL, CH, LI, SE, DK)

1. A pharmaceutical composition for external use and which has, as an active ingredient, a non-steroid anti-inflammatory compound (NSAI), characterised in that said active ingredient is a compound selected from:
i) 4-hydroxy-2-methyl-N-2-pyridinyl-2H-1,2-benzothiazine-3-carboxamid-1,1-dioxide(pyroxicam) of the formula: 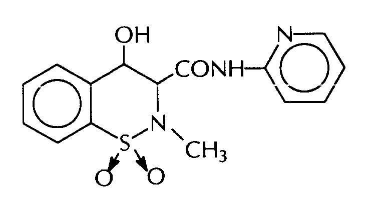
ii) alpha-(6-methoxy)naphthyl-propionic acid (naproxen) of the formula: 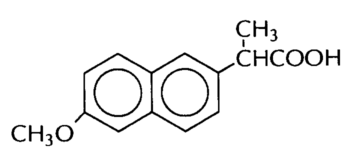
iii) alpha-(4-isobutyl)phenyl-propionic acid (ibuprofen) of the formula: 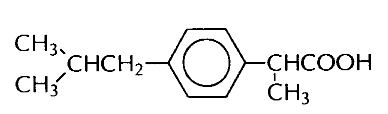
iv) a pharmaceutically acceptable salt of compound i), ii) or iii),
v) the diethylammonium salt of 2-(2,6-dichloroanilino)phenyl-acetic acid (diclofenac) of the formula: 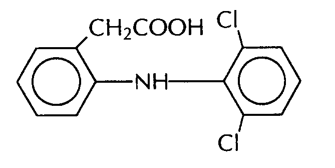 in that a pharmaceutically effective amount of said active ingredient is present in a pharmaceutically acceptable vehicle that includes, as a percutaneous penetration enhancer of said active ingredient, at least one of (C₆-C₁₈) fatty acids and alkyl and alkenyl esters thereof, and in that the pharmaceutical composition is a gel.

2. A composition as claimed in claim 1, characterised in that said ester is a (C₁-C₆) alkyl ester.

3. A composition as claimed in claim 2, characterised in that said ester is ispropyl myristate.

4. A composition as claimed in claim 1 or 2, characterised in that said fatty acid is oleic acid.

5. A composition as claimed in any preceding claim, characterised in that the concentration of the anti-inflammatory agent in the range of 0.01 and 50 wt%, the remainder being the vehicle.

6. A composition as claimed in any preceding claim, characterised in that the concentration of the percutaneous penetration enhancer is 0.1% of the total weight of the composition.

7. A composition as claimed in any preceding claim, wherein said NSAI in naproxen or ibuprofen.

8. A process for preparation of an anti-inflammatory composition as claimed in any preceding claim, comprising the step of mixing said active ingredient with said pharmaceutically acceptable vehicle to form a homogeneous gel.

## Claims (Claims for the following Contracting State(s): ES, GR)

1. A method of preparing a pharmaceutical composition for external use and which has, as an active ingredient, a non-steroid anti-inflammatory compound (NSAI), characterised in that said active ingredient is a compound selected from:
i) 4-hydroxy-2-methyl-N-2-pyridinyl-2H-1,2-benzothiazine-3-carboxamide-1,1-dioxide (pyroxicam) of the formula: 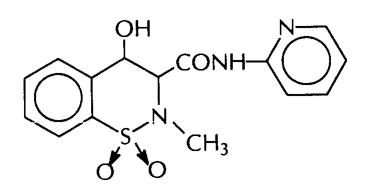
ii) alpha-(6-methoxy)naphthyl-propionic acid (naproxen) of the formula: 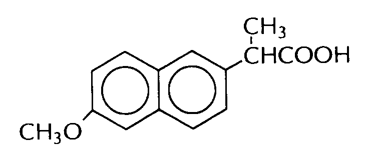
iii) alpha-(4-isobutyl)phenyl-propionic acid (ibuprofen) of the formula: 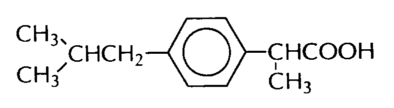
iv) a pharmaceutically acceptable salt of compound i), ii) or iii),
v) the diethylammonium salt of 2-(2,6-dichloroanilino)phenyl-acetic acid (diclofenac) of the formula: 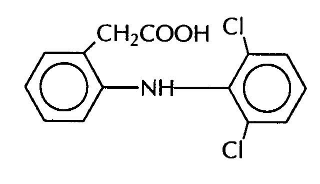 and in that a pharmaceutically effective amount of said active ingredient is mixed with a pharmaceutically acceptable vehicle that includes, as a percutaneous penetration enhancer of said active ingredient, at least one of (C₆-C₁₈) fatty acids and alkyl and alkenyl esters thereof, to form a homogeneous gel.

2. A method as claimed in claim 1, characterised in that said ester is a (C₁-C₆) alkyl ester.

3. A method as claimed in claim 2, characterised in that said ester is isopropyl myristate.

4. A method as claimed in claim 1 or 2, characterised in that said fatty acid is oleic acid.

5. A method as claimed in any preceding claim, characterised in that the concentration of the anti-inflammatory agent in the range of 0.01 and 50 wt%, the remainder being the vehicle.

6. A method as claimed in any preceding claim, characterised in that the concentration of the percutaneous penetration enhancer is 0.1% of the total weight of the composition.

7. A method as claimed in any preceding claim, wherein said NSAI is naproxen or ibuprofen.

## Patentansprüche (Patentansprüche für folgende(n) Vertragsstaat(en): AT, BE, DE, FR, GB, IT, LU, NL, CH, LI, SE, DK)

1. Pharmazeutische Zusammensetzung zur äußerlichen Anwendung, welche als wirksamen Bestandteil eine nicht-steroide entzündungshemmende Verbindung (NSAI) hat, **dadurch gekennzeichnet**, daß der wirksame Bestandteil eine Verbindung ist, die ausgewählt ist aus:
i) 4-Hydroxy-2-methyl-N-2-pyridinyl-2H-1,2-benzothiazin-3-carboxamid-1,1-dioxid (Pyroxicam) der Formel: 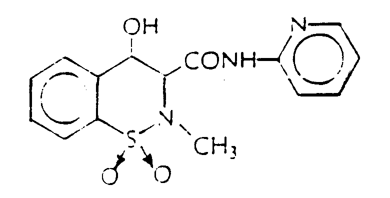
ii) alpha-(6-Methoxy)naphthyl-propionsäure (Naproxen) der Formel: 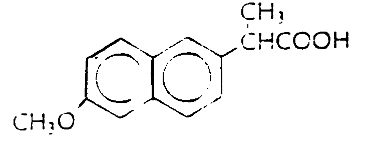
iii) alpha-(4-Isobutyl)phenyl-propionsäure (Ibuprofen) der Formel: 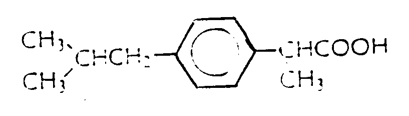
iv) einem pharmazeutisch annehmbaren Salz der Verbindung i), ii) oder iii),
v) dem Diethylammoniumsalz von 2-(2,6-Dichloranilino)-phenylessigsäure (Diclofenac) der Formel: 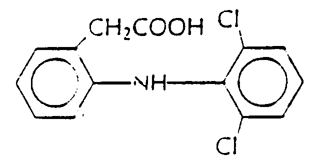 daß eine pharmazeutisch wirksame Menge des wirksamen Bestandteils in einem pharmazeutisch annehmbaren Vehikel vorhanden ist, welches als perkutanen Durchdringungsverstärker des wirksamen Bestandteils mindestens eine von (C₆-C₁₈)Fettsäuren und Alkyl- und Alkenylestern davon enthält, und daß die pharmazeutische Zusammensetzung ein Gel ist.

2. Zusammensetzung nach Anspruch 1, **dadurch gekennzeichnet**, daß der Ester ein (C₁-C₆)Alkylester ist.

3. Zusammensetzung nach Anspruch 2, **dadurch gekennzeichnet**, daß der Ester Isopropylmyristat ist.

4. Zusammensetzung nach Anspruch 1 oder 2, **dadurch gekennzeichnet**, daß die Fettsäure Ölsäure ist.

5. Zusammensetzung nach einem der vorhergehenden Ansprüche, **dadurch gekennzeichnet**, daß die Konzentration des entzündungshemmenden Mittels im Bereich von 0,01 und 50 Gew.-% liegt, wobei der Rest das Vehikel ist.

6. Zusammensetzung nach einem der vorhergehenden Ansprüche, **dadurch gekennzeichnet**, daß die Konzentration des perkutanen Durchdringungsverstärkers 0,1% des Gesamtgewichts der Zusammensetzung beträgt.

7. Zusammensetzung nach einem der vorhergehenden Ansprüche, worin das NSAI Naproxen oder Ibuprofen ist.

8. Verfahren zur Herstellung einer entzündungshemmenden Zusammensetzung nach einem der vorhergehenden Ansprüche, umfassend den Schritt des Vermischens des wirksamen Bestandteils mit dem pharmazeutisch annehmbaren Vehikel, um ein homogenes Gel zu bilden.

## Patentansprüche (Patentansprüche für folgende(n) Vertragsstaat(en): ES, GR)

1. Verfahren zum Herstellen einer pharmazeutischen Zusammensetzung zur äußerlichen Anwendung, welche als wirksamen Bestandteil eine nicht-steroide entzündungshemmende Verbindung (NSAI) hat, **dadurch gekennzeichnet**, daß der wirksame Bestandteil eine Verbindung ist, die ausgewählt ist aus:
i) 4-Hydroxy-2-methyl-N-2-pyridinyl-2H-1,2-benzothiazin-3-carboxamid-1,1-dioxid (Pyroxicam) der Formel: 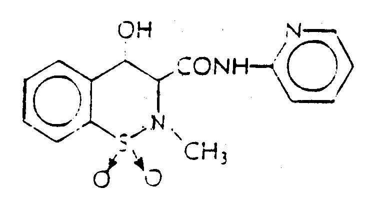
ii) alpha-(6-Methoxy)naphthyl-propionsäure (Naproxen) der Formel: 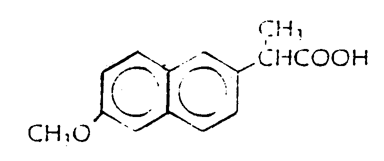
iii) alpha-(4-Isobutyl)phenyl-propionsäure (Ibuprofen) der Formel: 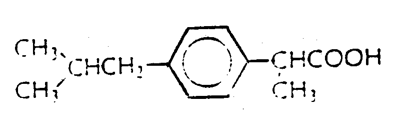
iv) einem pharmazeutisch annehmbaren Salz der Verbindung i), ii) oder iii),
v) dem Diethylammoniumsalz von 2-(2,6-Dichloranilino)-phenylessigsäure (Diclofenac) der Formel: 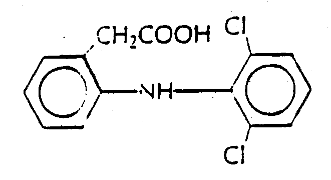 und daß eine pharmazeutisch wirksame Menge des wirksamen Bestandteils mit einem pharmazeutisch annehmbaren Vehikel vermischt wird, welches als perkutanen Durchdringungsverstärker des wirksamen Bestandteils mindestens eine von (c₆-C₁₈)Fettsäuren und Alkyl- und Alkenylestern davon enthält, um ein homogenes Gel zu bilden.

2. Verfahren nach Anspruch 1, **dadurch gekennzeichnet**, daß der Ester ein (C₁-C₆)Alkylester ist.

3. Verfahren nach Anspruch 2, **dadurch gekennzeichnet**, daß der Ester Isopropylmyristat ist.

4. Verfahren nach Anspruch 1 oder 2, **dadurch gekennzeichnet**, daß die Fettsäure Ölsäure ist.

5. Verfahren nach einem der vorhergehenden Ansprüche, **dadurch gekennzeichnet**, daß die Konzentration des entzündungshemmenden Mittels im Bereich von 0,01 und 50 Gew.-% liegt, wobei der Rest das Vehikel ist.

6. Verfahren nach einem der vorhergehenden Ansprüche, **dadurch gekennzeichnet**, daß die Konzentration des perkutanen Durchdringungsverstärkers 0,1% des Gesamtgewichts der Zusammensetzung beträgt.

7. Verfahren nach einem der vorhergehenden Ansprüche, worin das NSAI Naproxen oder Ibuprofen ist.

## Revendications (Revendications pour l'(les) Etat(s) contractant(s) suivant(s): AT, BE, DE, FR, GB, IT, LU, NL, CH, LI, SE, DK)

1. Composition pharmaceutique pour usage externe et qui a, comme ingrédient actif, un composé anti-inflammatoire non-stéroïdien (AINS), caractérisée en ce que ledit ingrédient actif est un composé choisi parmi :
i)4-hydroxy-2-méthyl-N-2-pyridinyl-2H-1,2-benzothiazine-3-carboxamide-1,1-dioxyde (pyroxicam) de formule : 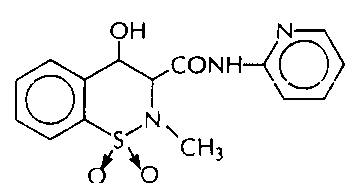
ii) acide a-(6-méthoxy)naphthyl-propionique (naproxène) de formule : 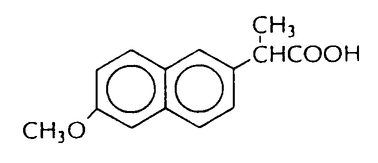
iii) acide a-(4-isobutyl)phényl-propionique (ibuprofène) de formule : 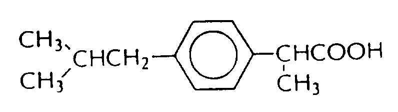
iv) un sel pharmaceutiquement acceptable du composé i), ii), ou iii),
v) le sel de diéthylammonium de l'acide 2-(2,6-dichloroanilino)phényl-acétique (diclofénac) de formule : 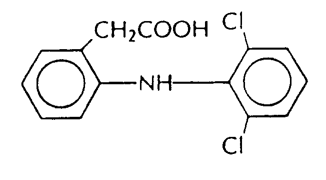 en ce qu'une quantité pharmaceutiquement efficace dudit ingrédient actif est présente dans un véhicule pharmaceutiquement acceptable qui comprend, comme stimulant de la pénétration percutanée dudit ingrédient actif, au moins un des acides gras en C₆-C₁₈ et des esters d'alkyle et d'alcényle de ceux-ci, et en ce que la composition pharmaceutique est un gel.

2. Composition telle que revendiquée à la revendication 1, caractérisée en ce que ledit ester est un ester d'alkyle en C₁-C₆.

3. Composition telle que revendiquée à la revendication 2, caractérisée en ce que ledit ester est le myristate d'isopropyle.

4. Composition telle que revendiquée à la revendication 1 ou 2, caractérisée en ce que ledit acide gras est l'acide oléique.

5. Composition telle que revendiquée dans l'une quelconque des revendications précédentes, caractérisée en ce que la concentration de l'agent anti-inflammatoire est dans la gamme allant de 0,01 à 50 % en poids, le reste étant le véhicule.

6. Composition telle que revendiquée dans l'une quelconque des revendications précédentes, caractérisée en ce que la concentration du stimulant de la pénétration percutanée est 0,1 % du poids total de la composition.

7. Composition telle que revendiquée dans l'une quelconque des revendications précédentes, dans laquelle ledit AINS est le naproxène ou l'ibuprofène.

8. Procédé pour la préparation d'une composition anti-inflammatoire telle que revendiquée dans l'une quelconque des revendications précédentes, comprenant l'étape consistant à mélanger ledit ingrédient actif avec ledit véhicule pharmaceutiquement acceptable pour former un gel homogène.

## Revendications (Revendications pour l'(les) Etat(s) contractant(s) suivant(s): ES, GR)

1. Méthode de préparation d'une composition pharmaceutique pour usage externe et qui a, comme ingrédient actif, un composé anti-inflammatoire non-stéroïdien (AINS), caractérisée en ce que ledit ingrédient actif est un composé choisi parmi :
i) 4-hydroxy-2-méthyl-N-2-pyridinyl-2H-1,2-benzothiazine-3-carboxamide-1,1-dioxyde (pyroxicam) de formule : 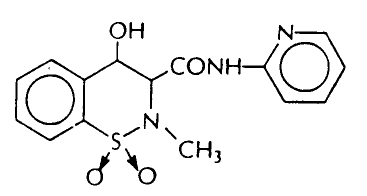
ii) acide a-(6-méthoxy)naphthyl-propionique (naproxène) de formule : 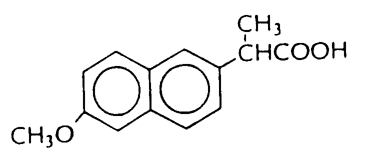
iii) acide a-(4-isobutyl)phényl-propionique (ibuprofène) de formule :
iv) un sel pharmaceutiquement acceptable du composé i), ii), ou iii),
v) le sel de diéthylammonium de l'acide 2-(2,6-dichloroanilino)phényl-acétique (diclofénac) de formule : 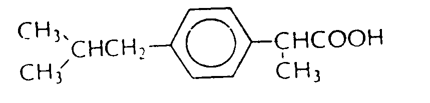 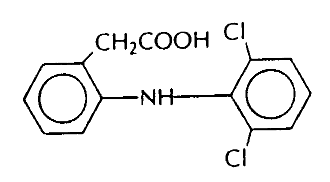 en ce qu'une quantité pharmaceutiquement efficace dudit ingrédient actif est mélangée avec un véhicule pharmaceutiquement acceptable qui comprend, comme stimulant de la pénétration percutanée dudit ingrédient actif, au moins un des acides gras en C₆-C₁₈ et des esters d'alkyle et d'alcényles de ceux-ci pour former un gel homogène.

2. Méthode telle que revendiquée à la revendication 1, caractérisée en ce que ledit ester est un ester d'alkyle en C₁-C₆.

3. Méthode telle que revendiquée à la revendication 2, caractérisée en ce que ledit ester est le myristate d'isopropyle.

4. Méthode telle que revendiquée à la revendication 1 ou 2, caractérisée en ce que ledit acide gras est l'acide oléique.

5. Méthode telle que revendiquée dans l'une quelconque des revendications précédentes, caractérisée en ce que la concentration de l'agent anti-inflammatoire est dans la gamme allant de 0,01 à 50 % en poids, le reste étant le véhicule.

6. Méthode telle que revendiquée dans l'une quelconque des revendications précédentes, caractérisée en ce que la concentration du stimulant de la pénétration percutanée est 0,1 % du poids total de la composition.

7. Méthode telle que revendiquée dans l'une quelconque des revendications précédentes, dans laquelle ledit AINS est le naproxène ou l'ibuprofène.
